# EUROPEAN PATENT APPLICATION

(11) **EP 1 820 531 A1**
(43) Date of publication of application: **22.08.2007**
(21) Application number: 05811245.9
(22) Date of filing: 01.12.2005
(51) Int. Cl.: A61M 25/00

(54) **THROMBUS SUCTION CATHETER WITH EXCELLENT PASSABLENESS**

(30) Priority: 07.12.2004 JP 2004353474
(71) Applicant: NIPRO CORPORATION, Kita-ku, Osaka-shi, Osaka-fu, 531-8510 (JP)
(72) Inventor: Mori, Satoru, Kita-ku, Osaka-shi, Osaka 5318510 (JP)
(74) Representative: Setna, Rohan P.
(86) International application number: PCT/JP2005/022059
(87) International publication number: WO 2006/062023

(57) **Abstract**

To provide a thrombus suction catheter with excellent crossability, capable of suctioning atheroma, reaching a sharply-curved affected area and passing through a narrowed section of the blood vessel.

A thrombus suction catheter is a tubular device comprising a catheter body (1) with a thrombus suction lumen (11) and a guide wire lumen (13); and a connector (2) provided on a proximal end of the catheter body. The thrombus suction lumen 11 has a suction port (12) in a sidewall of a tip of the catheter body and passes through the catheter body to a proximal end of the catheter body, while the guide wire lumen (13) has a guide wire insertion port (14) in the sidewall of the tip and passes through the tip along a longitudinal axis of the catheter body. The suction port 12 and the guide wire insertion port 14 are formed on the sides opposite to one another with respect to the longitudinal axis of the catheter body.

## Description

### Technical Field

The present invention relates to a thrombus suction catheter suitable for removal of coronary thrombus, which makes it possible to remove atheroma by suction and has a considerably improved crossability.

### Background Art

An acute myocardial infarction is a disease in which the heart loses its function due to interruption of blood supply to the heart caused by the fact that the blood vessels (coronary arteries) carrying oxygen and nutrients to the heart are occluded by blood clots formed therein. In Japan, about forty thousands (40,000) persons are being treated annually. The heart disease is the leading cause of death in Europe and the United States and the heart disease in Japan has risen to the second leading cause of death because of change in the food culture to Western-style or increasing stress in the social life, following the malignant neoplasm (cancers). Recently, the dead rate in hospitals is lowered to less than 10 percent by reperfusion treatment to recanalize the occluded blood vessels. Generally used reperfusion treatment includes a method of injecting a thrombolytic agent into the vessel to dissolve the blood clots, and a method of expanding a blood vessel narrowed by thrombus with a percutaneous transluminal coronary angioplasty (PTCA) balloon catheter.

The coronary thrombus is considered to be inspired by infiltration of low density lipoprotein (LDL) through intercellular clearances that are formed between intimal (vascular endothelial) cells that constitute the blood vessel, by factors such as stress, change in food culture to Western-style and conventionalized smoking or drinking.

The infiltrated LDL undergoes oxidation to form oxidized LDL, and thus macrophages converge on and begin to phagocytize the oxidized LDL that is a foreign substance in the living body to remove the same. As the result, collection of a gruel-like substance called "LIPIDO CORE" between vascular endothelium and media is taken place to quaquaversally expand within the blood vessel, resulting in formation of atheroma. If the collection of LIPIDO CORE exceeds the limit of a certain allowable range, it results in rupture of the vascular endothelium at the area of atheroma. Consequently the platelets in the blood form clots to repair the ruptured area of the blood vessel. The formation of the blood clots causes block of the blood flow in the coronary artery and prevents the heart from being supplied with oxygen and nutrients, resulting in loss of the heart function. Failure in reperfusion by early removal of the formed blood clots leads to the death.

The thrombus suction therapy (suction therapy) is a treatment procedure in which a fine tube with a diameter of about 1.5 mm, called as a catheter, is inserted through a leg or arm and into the coronary artery, navigated into an affected area in the coronary artery and then operated to remove the actual thrombus by suctioning. In the thrombus suction therapy, the thrombus per se that is the cause of narrowing of blood vessels is removed, thus making it possible to avoid risks associated with conventional procedures, for example, coronary restenosis due to the thrombi that can not be dissolved by medicines, damage due to excessive dilatation of the blood vessel, and the like.

The catheter for removing blood clots in coronary artery is called as a thrombus suction catheter, and used in combination with a suction device. The thrombus suction catheter is required to have a certain degree of mechanical strength to prevent the wall of the catheter from being collapsed under the negative pressure, as well as to have an area of an open section (opening area) as large as possible to make it easy to discharge the aspirate from a lesion to the outside of the catheter body. The conventional catheter has an opening area of about 0.65 mm² and a wall thickness of not less than 0.15 mm to maintain its strength. When the catheter is increased in wall thickness to withstand the collapse, the catheter would lose its flexibility, thus making it impossible to navigate the catheter into the affected area in a sharply-curved portion such as #3 or #4 of the coronary artery because of its poor trackability.

Furthermore, another important factor of the thrombus suction catheter is a tip shape of the catheter.

The conventional thrombus suction catheter has an opening of the tip slanted at an angle of about 30 to 45 degrees to the longitudinal axis, and a guide wire lumen provided on the tube wall on the side opposite to the slant plane of the opening (e.g., see patent document 1). Thus, the axis of the guide wire lumen is greatly misaligned from that of the thrombus suction lumen (located on the same axis as the catheter). When inserting the catheter into the vessel to allow its tip to pass through the narrowed section of the blood vessel, the catheter is navigated over the preceding guide wire and thus the slanted plane of the catheter may run into the narrowed section of the blood vessel to prevent the catheter from being advanced.
Patent document 1: US Patent No. 5,827,229 (Fig. 5(a), Fig. 6(d))

The inventors have therefore proposed a thrombus suction catheter improved in both suctioning performance and crossability, which enables to aspirate the atheroma and is improved in trackability so that it is able to reach a sharply-curved, affected area (cf. Japanese patent application serial No. 2003-294616).

The aforesaid catheter is improved in both suctioning performance and crossability, but it is inadequate for application to the affected areas such as, e.g., narrowed sections of the blood vessels that require more improved crossability because of a lack of ability to transmit a force applied to the proximal end of the catheter to the distal end thereof (pushability). This results from the facts that the catheter has been increased in opening area to improve the suctioning performance and it has been increased in flexibility and thus in trackability to improve the crossability.

### Disclosure of Invention

### Technical Problems to be solved by the invention

The present invention has been made in view of the above circumstances and designed to provide a thrombus suction catheter with excellent crossability, which enables to perform suctioning of atheroma, arrival to any sharply-curved affected area and passage of narrowed sections of blood vessels.

The thrombus suction catheter according to the present invention is a tubular device comprising a catheter body with a thrombus suction lumen and a guide wire lumen, and a connector provided on a proximal end of the catheter body, said thrombus suction lumen having a suction port in a sidewall of a tip division of the catheter body and passing through the catheter body to a proximal end of the catheter body, while said guide wire lumen having a guide wire insertion port in a sidewall of the tip division and passing through the distal end of the tip division on a longitudinal axis of the catheter body, said suction port and said guide wire insertion port being provided on the sides opposite to one another with respect to the longitudinal axis of the catheter body.

The guide wire insertion port is preferably provided at a position longitudinally shifted to the proximal side of the catheter body from the position opposed to the suction port. In this case, the guide wire lumen is preferably not too long in length to prevent the catheter body from being decreased in flexibility. More specifically, the guide wire lumen is preferably so designed that the difference (L1-L2) between a longitudinal length L1 from the distal end of the tip to the guide wire insertion port and a longitudinal length L2 from the distal end of the tip to the proximal edge of the suction port is not less than 3 mm but not more than 15 mm.

In order to prevent the suction port from sticking fast to the wall of blood vessel while keeping its sufficient suctioning effect, the catheter body may be provided with a side hole, which has a diameter sufficiently smaller than that of the suction port and is communicated with the thrombus suction lumen, in the sidewall of the catheter body close to the guide wire insertion port on the same side as the guide wire insertion port. This allows the catheter body to suction the thrombus through the side hole while keeping the sufficient suction power of the suction port. Also, the catheter body may be provided with a marker on the distal side close to the suction port to check the insert location of the catheter.

The present invention has generally been described as above, the present invention will be further understood from the following description, taken in conjunction with some specific embodiments thereof. However, these embodiments are given by way of illustration only and thus are not limitative of the present invention unless otherwise stated.

### Effects of the Invention

The present invention would have the following advantages:
1) The suction port is formed in the sidewall of the tip and can be placed over the atheroma formed in the wall of blood vessel, thus making it possible to produce a large suction power at the time of suctioning, which in turn makes it possible to suction the atheroma. 2) The guide wire lumen is being passed through the distal end of the tip on the longitudinal axis of the catheter body so that the guide wire is positioned on the longitudinal axis of the catheter body when navigating the catheter over the guide wire, thus making it possible to allow the catheter to smoothly go through the narrowed part of the blood vessel. 3) The guide wire lumen is provided in the part extending from the proximal side of the tip close to the suction port to the distal end of the tip, thus making it possible to reduce the outer diameter of the distal side of the catheter, which in turn makes it possible to improve the crossability.

### Brief Description of Drawings

Fig. 1 is a plan view of a thrombus suction catheter according to one embodiment of the present invention;
Fig. 2 is an enlarged plan view of a part of the catheter shown in Fig. 1 including a suction port of the catheter;
Fig. 3 is a vertical longitudinal sectional view of Fig. 2;
Fig. 4 is a cross section view of the catheter along the line X-X in Fig.2;
Fig. 5 is an enlarged vertical longitudinal sectional view of a part including a suction port of a catheter according to another embodiment of the present invention;
Fig. 6 is a vertical longitudinal sectional view of a thrombus suction catheter of the prior art;
Fig. 7 is a vertical longitudinal sectional view of another thrombus suction catheter of the prior art; and
Fig. 8 is a schematic diagram of performance test.

### Description of reference symbols

1: catheter body
11: thrombus suction lumen
12: suction port
13: guide wire lumen
14: guide wire insertion port
15: side hole
16: (angiographic) marker
2: connector

### Best Mode for Carrying Out the Invention

Set L1 - L2 = 5 mm, and the catheter is provided with a marker for checking the insert location of the catheter on the distal side of the tip division close to the suction port.

### Embodiment 1

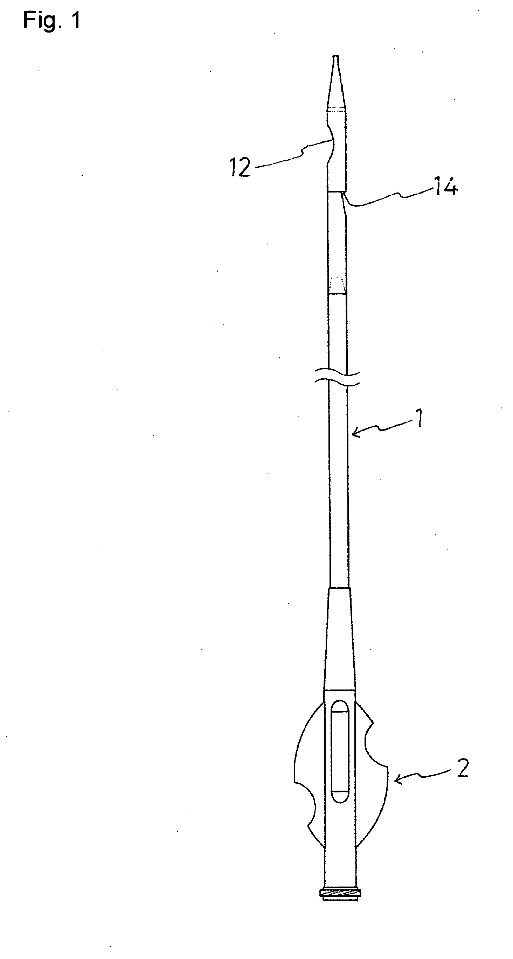

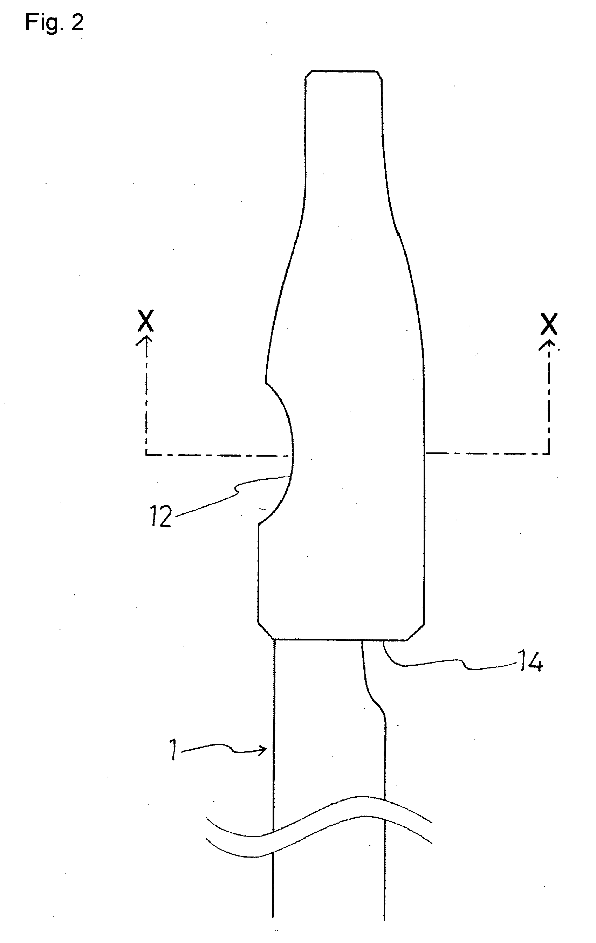

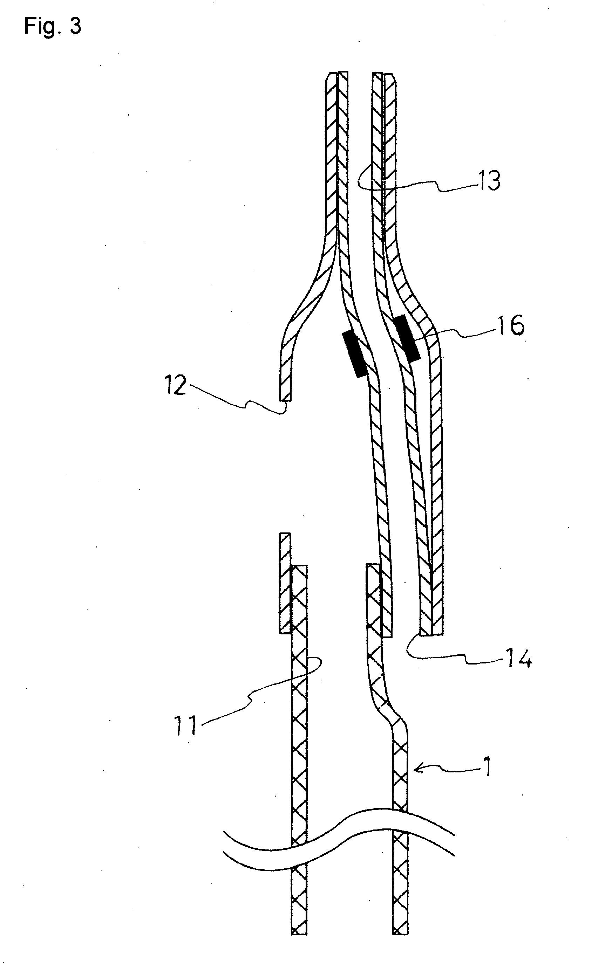

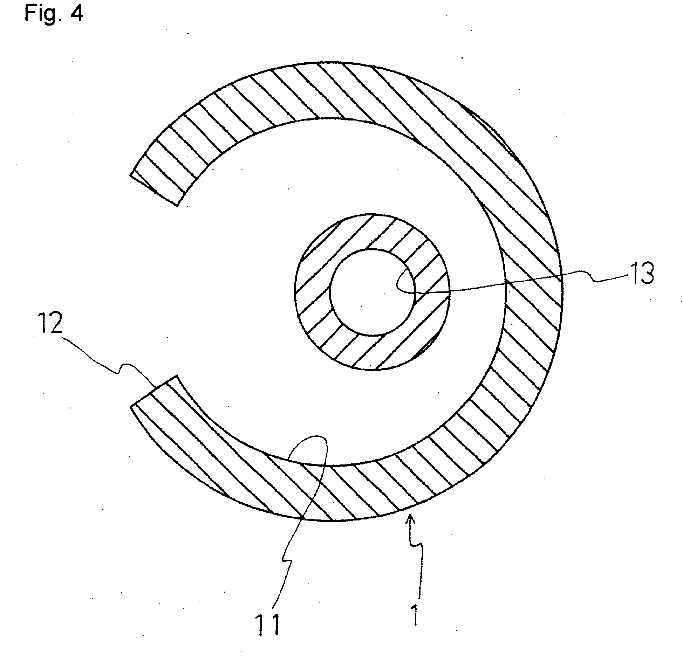

Firstly, embodiment 1 will be explained with reference to Figs. 1 to 3. Fig. 1 is a plan view of embodiment 1 of the present invention; Fig. 2 is an enlarged plan view of a part including a suction port shown in Fig. 1; Fig. 3 is an enlarged vertical sectional view of the part shown in Fig. 2; and Fig. 4 is an enlarged cross sectional view taken along the line X-X in Fig. 1.

As illustrated in Figs. 1 to 4, a thrombus suction catheter of embodiment 1 is a tubular device comprising a catheter body 1 with a thrombus suction lumen 11 and a guide wire lumen 13, and a connector 2 provided at a proximal end of the catheter body 1. The thrombus suction lumen 11 has a suction port 12 formed in a sidewall of a distal portion of the catheter body and passes through a proximal end of the catheter body. The guide wire lumen 13 has a guide wire insertion port 14 formed in a sidewall of the distal portion of the catheter and passes through a distal end of the catheter body along a longitudinal axis of the catheter body. The suction port 12 and the guide wire insertion port 14 are located on the opposite sides of the longitudinal axis of the catheter body. The guide wire insertion port is provided at a position longitudinally shifted to the proximal side of the catheter body from the position opposed to the suction port.

The catheter body 1 is a tubular member made of flexible plastic such as polyamide elastomer, polyurethane, polyester elastomer and polyethylene, and provided with the thrombus suction lumen 11 and the guide wire lumen 13. The thrombus suction lumen 11 extends from the proximal end of the catheter to the tip division of the catheter body and opens into the suction port provided in the sidewall of the tip division of the catheter body. The guide wire lumen 13 starts from the guide wire insertion port 14 provided in the side wall of the distal portion of the catheter body to the distal end of the catheter body and passes through the distal end on the longitudinal axis of the catheter body. The suction port 12 and the guide wire insertion port 14 are provided on the opposite side of the longitudinal axis of the catheter body.
In the thrombus suction catheter of the present invention, the suction port 12 is provided in the side wall of the tip division, and thus there is a fear that the catheter is prevented from being moved forward due to accordion-folded deformation of catheter which may occur at a weakened area around the suction port 12 when pushing the catheter forward. To solve this problem, the present invention uses a structure in which a portion of the catheter that forms the suction port 12 is reinforced by the guide wire. The guide wire insertion port 14 is preferably provided at a position longitudinally shifted to the proximal side of the catheter body from the position opposed to the suction port 12. In this case, the guide wire lumen 13 is preferably not too long in length to maintain the flexibility of the catheter body 1 and, preferably, the difference (L1-L2) between a longitudinal distance L1 from the distal end of the catheter body 1 to the guide wire insertion port 14 and a longitudinal distance L2 from the distal end of the catheter body to the proximal edge of the suction port 12 meets the equation: 3 mm ≤ (L1-L2) ≤ 15 mm.

The thrombus suction catheter may be provided with a reinforcing wire lumen (not illustrated in the drawings), which extends from the proximal end to the distal end of the catheter, to improve the ability of the catheter to transmit the force pushing the catheter forward to the distal end of the catheter (called "pushability"). In this case, the reinforcing wire lumen is filled with a reinforcing wire made of stainless steel.

The tip division of the catheter includes the suction port 12 and the distal end of the tip division is located on the longitudinal axis of the catheter as illustrated in Figs. 2 and 3. The guide wire lumen 13 starts from the guide wire insertion port 14, which is provided at a position longitudinally shifted to the proximal side of the catheter body from the position opposed to the suction port 12, extends in the direction of the distal end and opens at the distal end of the dip division on the longitudinal axis of the catheter.

The thrombus suction lumen 11 is a passage for thrombi sucked into the suction port 12 of the tip division, and the thrombi are collected in a thrombus collection bottle (not illustrated in the drawings) through the lumen 11. The suction port 12 is provided in the side wall of the tip division, so that it may cling to the swelled atheroma when placing the suction port over the atheroma and suctioning the atheroma with a suction pump (not illustrated in the drawings), thus making it possible to considerably increase the suction power of the catheter (the suction power of the suction port would be approximately equal to an actual pressure of the pump, provided that the suction port stuck fast to the atheroma.), which in turn allows the catheter to suction lipid cores present in the vascular endothelium.

The thrombus suction catheter of the present invention may be provided with a marker (angiographic marker) 16 on the distal side of the catheter adjacent to or extremely close to the suction port 12 to check the insert location of the catheter. The angiographic marker 16 may be formed by a method of molding the tip after kneading a contrast agent into the area on the distal portion of the suction port 12, or a method of providing a ring of platiniridium on the tip division by press fitting or the like.

The above catheter makes it possible to suction the atheroma as the suction port is provided in the side wall. Further, the catheter is capable of smoothly passing through the narrowed portion of the blood vessel since the guide wire lumen is being passed through the distal end of the tip division on the longitudinal axis of the catheter body. In addition, the catheter can be improved in crossability by reducing the outer diameter of the catheter body since the guide wire lumen is provided so that it extends from the position close to the proximal edge of the suction port on the proximal side of the tip division to the distal end of the tip division.

### Embodiment 2

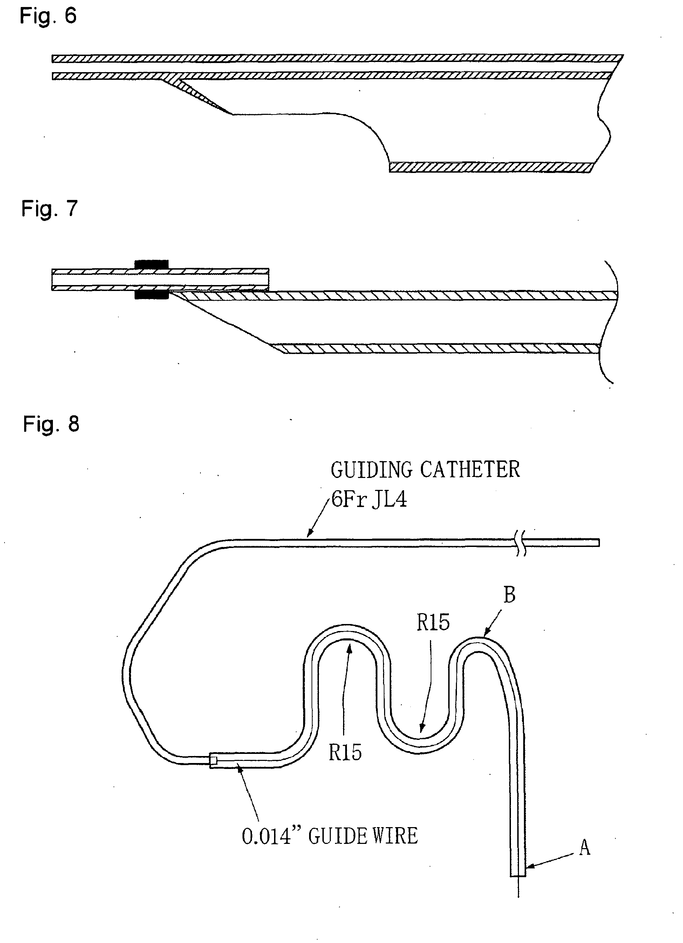

Embodiment 2 of the present invention will be explained below with reference to Fig. 7.

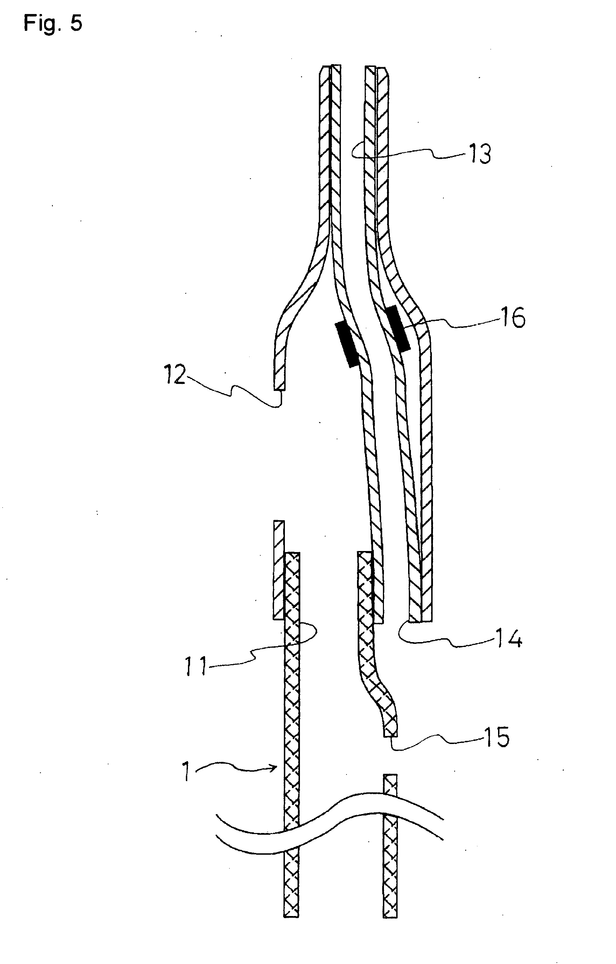

As illustrated in Fig. 5, the thrombus suction catheter of Embodiment 2 is a modified form of Embodiment 1, in which the catheter body is provided with a side hole 15 in the side wall on the same side as the guide wire insertion port 14 at a position close to the guide wire insertion port 14. The side hole 15 has a diameter sufficiently smaller than that of the suction port 12 and communicates with the thrombus suction lumen 11. This enables to suction the thrombus through the side hole 15 when carrying out suction with a suction pump or the like, while allowing the suction port 12 to keep the sufficient suction power.

The thrombus suction catheter of this embodiment is somewhat inferior in suction power to that of embodiment 1, but it enables to suction the thrombus around the catheter evenly as the suction is also performed through the side hole 15. Further, even if the suction port 12 stuck to the vessel wall, the suction is carried out through the sidewall 15, thus making it possible to prevent the suction port 12 from sticking fast to the vessel wall.

### PERFORMANCE TEST

For thrombus suction catheters listed in Table 1, comparison tests were made on their crossability and flexibility. Results are shown in Table 2.

The performance tests were carried out by the steps of preparing a simulated blood vessel model (length 350 mm; inner diameter 4.0 mm (semicircle), waveform with cycle R 15 mm) with a guiding catheter (made by Boston Scientific Corporation MODEL-6Fr JL4: Inner diameter 0.070") inserted therein as shown in Fig. 8, submerging the simulated vessel model in warm water of 37 degree centigrade, inserting a guide wire (made by ACS, HI-TORQUE BALANCE MIDDLEWEIGHT, 0.014") into the guiding catheter, introducing the thrombus suction catheter into the simulated blood vessel model along the guide wire, and performing measurement of the maximum length of the catheter inserted into the simulated blood vessel model.

The thrombus suction catheters (N=10) prepared in embodiments 1 and 2 could be inserted to the position B (insertion length 35 cm) without kinking, while the thrombus suction catheters of both relative embodiment 1 (N=10) and relative embodiment 2 (N=2) could be inserted only to the position A because of their kinking (inserted length 20 cm). The thrombus suction catheters of embodiments 1 and 2 have an outer diameter of 3.9 Fr (equivalent to an outer diameter of 1.30 mm), cross-section area of lumen: 0.95 mm² on the proximal side from the guide wire insertion port, 0.79 mm² on the distal die from the guide wire insertion.

The thrombus suction catheters of comparative embodiment 1 have distal outer diameter 4.5 Fr, proximal outer diameter 3.9 Fr, cross-section area of lumen: 0.65 mm² on the proximal side from the guide wire insertion port, 0.90 mm² on the distal side from the guide wire insertion port. The thrombus suction catheters of comparative embodiment 2 have outer diameter 3.9 Fr, cross-section area of lumen: 0.95 mm² on the proximal side from the guide wire insertion port, 0.79 mm² on the distal side from the guide wire insertion port.

The results revealed that the thrombus suction catheter of the present invention is much improved in crossability and in flexibility as compared with commercially available thrombus suction catheters.

**TABLE 1**

| | Remarks |
|---|---|
| Embodiment 1 | Fig. 1 (L1-L2=5 mm, A tip is provided with a marker for checking the inserted position on the distal side close to the suction port.) |
| Embodiment 2 | Fig. 5 (In the catheter of Fig. 1, a side hole is provided at a position away from the guide wire insertion port toward the distal side by 2 mm.) |
| Comparative Embodiment 1 | Fig. 6 (A cut surface is at least partly concaved in its slanted direction and the distal side of the cut surface is flattened and made into flexible.) |
| Comparative Embodiment 2 | Fig. 7 (A main shaft is cut at an angle toward the proximal side and a distal end of a guide wire shaft is protruded forward from the main shaft.) |

**TABLE 2**

| | Flexibility | Crossability |
|---|---|---|
| Embodiment 1 | ⊚ | ⊚ |
| Embodiment 2 | ⊚ | ⊚ |
| Comparative Embodiment 1 | ○ | ○ |
| Comparative Embodiment 2 | ○ | ○ |

| | | |
|---|---|---|
| (Remarks) ⊚: very good, ○; good | | |

## Claims

1. A thrombus suction catheter with excellent crossability, composed of a tubular device, which comprises a catheter body with a thrombus suction lumen and a guide wire lumen, and a connector provided on a proximal end of the catheter body, said thrombus suction lumen having a suction port in a sidewall of a tip division of the catheter body and passing through the catheter body to a proximal end of the catheter body, while said guide wire lumen having a guide wire insertion port in a sidewall of the tip division and passing through the distal end of the tip division on a longitudinal axis of the catheter body, said suction port and said guide wire insertion port being provided on the sides opposite to one another with respect to the longitudinal axis of the catheter body.

2. The thrombus suction catheter according to claim 1, wherein said guide wire insertion port is provided at a position longitudinally shifted to the proximal side of the catheter body from the position opposed to the suction port.

3. The thrombus suction catheter according to claim 2, wherein the difference (L1-L2) between a longitudinal length L1 from the distal end of the tip to the guide wire insertion port and a longitudinal length L2 from the distal end of the tip to the proximal edge of the suction port is not less than 3 mm but not more than 15 mm.

4. The thrombus suction catheter according to any one of preceding claims 1 to 4, wherein the catheter body is provided with a side hole, which has a diameter sufficiently smaller than that of the suction port and is communicated with the thrombus suction lumen, in the sidewall of the catheter body close to the guide wire insertion port on the same side as the guide wire insertion port.

5. The thrombus suction catheter according to any one of claims 1 to 4, wherein the catheter body is provided with a marker for checking the insert location of the catheter at a position close to the suction port on the distal side of the tip division.
